# EUROPEAN PATENT SPECIFICATION

(11) **EP 1 198 455 B1**
(45) Date of publication and mention of the grant of the patent: **10.12.2003**
(21) Application number: 00951393.8
(22) Date of filing: 17.07.2000
(51) Int. Cl.: C07D 213/50

(54) **PROCESS FOR PREPARING 1-(6-METHYLPYRIDIN-3-YL)-2- (4-(METHYLSULPHONYL) PHENYL] ETHANONE**
VERFAHREN ZUR HERSTELLUNG VON 1-(6-METHYLPYRIDIN-3-YL)-2-[(4-(METHYLSULFONYL)PHENYL] ETHANON
PROCEDE DE PREPARATION DE 1-(6-METHYLPYRIDIN-3-YL)-2- 4-(METHYLSULFONYL) PHENYL] ETHANONE

(30) Priority: 27.07.1999 EP 99114667; 03.03.2000 US 186680 P
(43) Date of publication of application: 24.04.2002
(73) Proprietor: Lonza AG, 4052 Basel (CH); Merck & Co., Inc., Rahway, NJ 07065-0907 (US)
(72) Inventor: BESSARD, Yves, CH-3960 Sierre (CH); LERESCHE, James, Edward, CH-3930 Visp (CH)
(74) Representative: Ritthaler, Wolfgang, Dr.rer.nat.Dipl.-Chem.
(86) International application number: EP0006825
(87) International publication number: WO01007410

(56) References cited:
- EP-A- 0 062 238
- WO-A-98/47871
- WO-A-99/15503
- US-A- 3 717 647
- US-A- 4 115 578
- BURGER A. & WALTER JR., C.R.: "Some .alpha.-substituted .beta.-pyridylethylamines" THE JOURNAL OF THE AMERICAN CHEMICAL SOCIETY, vol. 72, no. 5, May 1950 (1950-05), pages 1988-1990, XP002128997

## Description

The invention encompasses a novel process for preparing 1-(6-methylpyridin-3-yl)-2-[(4-(methylsulphonyl)phenyl]ethanone of the formula 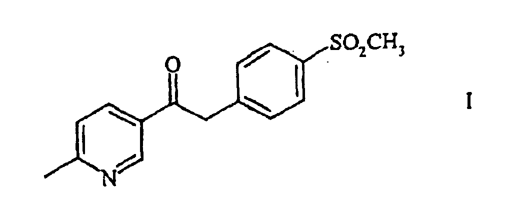 1-(6-methylpyridin-3-yl)-2-[(4-(methylsulphonyl)phenyl]ethanone is an important intermediate for preparing so-called COX-2 inhibitors, pharmaceutically active compounds having analgesic and antiinflammatory action (R.S. Friesen et al., Bioorganic & Medicinal Chemistry Letters 8 (1998) 2777-2782; WO 98/03484).

The object of the invention was to provide a technically feasible process for preparing the intermediate of the formula I.
This object was achieved by the novel process according to Claim 1.

The process according to the invention is characterized by five steps, where,
in the first step a), 4-(methylthio)benzyl alcohol is converted with hydrochloric acid into 4-(methylthio)benzyl chloride,
in the second step b), 4-(methylthio)benzyl chloride is converted with an alkali metal cyanide into 4-(methylthio)phenylacetonitrile,
in the third step c), 4-(methylthio)phenylacetonitrile is condensed with a 6-methylnicotinic ester to give 3-[2-(4-(methylthio)phenyl)-2-cyanoacetyl](6-methyl)pyridine of the formula 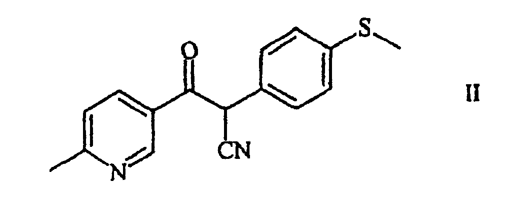 in the fourth step d), 3-[2-(4-(methylthio)phenyl)-2-cyanoacetyl](6-methyl)pyridine is hydrolysed and decarboxylated under acidic conditions to give 3-[2-(4-(methylthio)phenyl)acetyl](6-methyl)pyridine of the formula 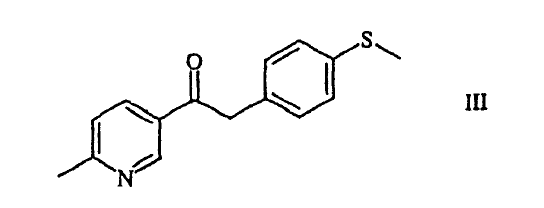 and, in the last step e), 3-[2-(4-(methylthio)phenyl)acetyl](6-methyl)pyridine is oxidized to give the end product.

### Step a:

The chlorination of 4-(methylthio)benzyl alcohol to 4-(methylthio)benzyl chloride is carried out using hydrochloric acid, advantageously using concentrated hydrochloric acid, at a temperature of from 10°C to 40°C.

The reaction is usually carried out in an organic solvent, advantageously in a water-immiscible solvent, such as, for example, in toluene.

Typically, the chlorination takes about 1 h to 4 h. The 4-(methylthio)benzyl chloride can be obtained in a simple manner by neutralizing the organic phase and removing the solvent. Further purification can be achieved by distillation.

### Step b:

The cyanidation of 4-(methylthio)benzyl chloride is carried out using an alkali metal cyanide, advantageously in the presence of a phase transfer catalyst.
Suitable alkali metal cyanides are sodium cyanide or potassium cyanide.
The phase transfer catalysts which can be chosen are known in the art. Suitable are tetraalkylammonium halides, such as, for example, tetra-n-butylammonium chloride or tetra-n-butylammonium bromide.
In general, the reaction is carried out in the presence of a water-immiscible solvent, such as, for example, toluene; if appropriate, water can be added.
The reaction temperature is advantageously from 60°C to 100°C.
After a reaction time of 1 h to 6 h, the product can be isolated in a simple manner from the organic phase by removing the solvent.
Further purification of the product can be achieved by recrystallization from, for example, diisopropyl ether.

### Step c:

In the third step, ((methylthio)phenyl)acetonitrile is condensed with a 6-methylnicotinic ester to give 3-[2-(4-(methylthio)phenyl)-2-cyanoacetyl](6-methyl)pyridine of the formula

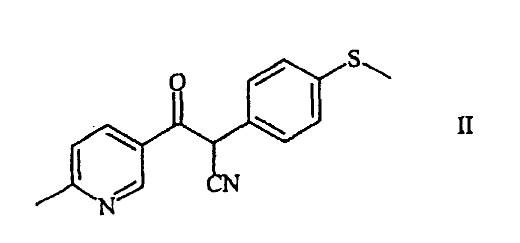

The condensation is advantageously carried out in the presence of an alkali metal alkoxide, at a temperature between 60°C and 110°C.
Suitable alkali metal alkoxides are, for example, sodium methoxide or potassium tert-butoxide. The reaction is advantageously carried out in the presence of a lower alcohol or an aromatic hydrocarbon as solvent.
After the condensation, the 3-[2-(4-(methylthio)phenyl)-2-cyanoacetyl](6-methyl)pyridine can be obtained, for example, by adding the reaction mixture to cold water and precipitating the product from the aqueous phase by acidifying it slightly.

### Step d:

Hydrolysis and decarboxylation to give 3-[2-(4-(methylthio)phenyl)acetyl](6-methyl)pyridine of the formula 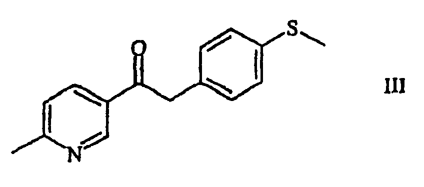 are carried out under acidic conditions.
Suitable acids are hydrochloric acid, phosphoric acid or mixtures of acetic acid with a mineral acid.
Advantageously, a mixture of acetic acid and a mineral acid is employed, at a temperature of from 50°C to 115°C.

Particular preference is given to mixtures of acetic acid with concentrated hydrochloric acid or mixtures of acetic acid with concentrated sulphuric acid. If appropriate, a certain amount of water can be added to the mixtures.
Good results were obtained using mixtures of acetic acid/concentrated hydrochloric acid 1:3 or acetic acid/concentrated sulphuric acid/water 1:1:1.
After a reaction time of about 1 h to 20 h, the mixture can be neutralized using, for example, an aqueous ammonia solution, as a result of which the product precipitates out and can be isolated in a simple manner.

### Step e:

Oxidation of 3-[2-(4-(methylthio)phenyl)acetyl](6-methyl)pyridine to the end product is advantageously carried out using hydrogen peroxide in the presence of an alkali metal tungstate, at a temperature of from 10°C to 40°C, preferably at about 20°C.
A particularly suitable alkali metal tungstate is sodium tungstate of the formula Na₂WO₁.2H₂O. The alkali metal tungstate is generally employed in catalytic amounts of from 0.5 mol% to 20 mol%, based on the 3-[2-(4-(methylthio)phenyl)acetyl](6-methyl)pyridine used. The reaction is advantageously carried out in the presence of a lower alcohol as solvent. After a reaction time of about 1 h to 6 h, the end product can be precipitated out by addition of water and then be isolated without any problems.

### Examples

### Preparation of 4-(methylthio)benzyl chloride

Under an atmosphere of nitrogen, 78.7 g (500 mmo)l [sic] of 4-(methylthio)benzyl alcohol were dissolved in 154.5 g of toluene. 131.6 g, (1.3 mol) of cone. HCl were added, and the mixture was stirred at 20-25°C for 30 min. After 2 h (no starting material left according to TLC), the reaction mixture was diluted with 349 g of toluene and the aqueous phase was separated off. The organic phase was neutralized using 14.0 g of NaHCO₃ and, after 15 min, filtered, and the solvent was evaporated. The residue that remained consisted of 107.4 g of a yellow oil with toluene, corresponding to a yield of >95% (according to NMR).
- ¹H-NMR (CDCl₃) :: 7.30 (2H, d) ;
7.22 (2H,d);
4.55 (2H, s);
2.47 (3H, s).
- ¹H-NMR (DMSO):: 7.37 (2H, d) ;
7.25 (2H, d);
4.73 (2H, d) ;
2.47 (3H,s).

### Preparation of 4-(methylthio)phenylacetonitrile

Under an atmosphere of nitrogen, 25.9 g (150 mmol) of 4-(methylthio)benzyl chloride were dissolved in 45.5 g of toluene. 9.29 g (180 mmol) of sodium cyanide, 0.92 g (2.9 mmol) of tetrabutylammonium chloride and 14.4 g of water were then added. The mixture was stirred at 80-85°C for 2 h. The reaction mixture was admixed with 30 g of toluene and 45 g of water, the aqueous phase was decanted off and the organic phase was concentrated. This gave a residue of 24.6 g of the title product in a yield of >95% (according to NMR) in the form of a pink solid.
- ¹H-NMR (CDCl₃) :: 7.25 (4H,m);
3.70 (2H,s);
2.47 (3H,s).

### Preparation of 3-[2-(4-(methylthio)phenyl)-2-cyanoacetyl](6-methyl)pyridine

Under an atmosphere of nitrogen, a mixture of 38.5 g (250 mmol) of ethyl 6-methylnicotinate, 29.9 g (500 mmol) of sodium methoxide (90.5%) and 300 ml of toluene was added, at 85-90°C and over the course of 30 min, to a solution of 47.3 g (250 mmol) of 4-(methylthio)phenylacetonitrile in 75 ml of toluene. This mixture was stirred under reflux for 14 h, then distilled until the overhead temperature exceeded 110°C and kept at reflux for another 6 h. The reaction mixture was poured into 500 g of ice water, the organic phase was decanted off and the aqueous phase was extracted with 3 × 100 ml of toluene. The aqueous phase was acidified to pH 6.0 using conc. HCl. The resulting yellow-beige suspension was filtered and the residue was washed with water and dried. This gave 53.9 g (76%) of the title product in the form of a yellow solid.
- ¹H-NMR (CDCl₃) :: 9.00 (1H,s);
8.10 (1H,d);
7.3 (5H,m);
5.45 (1H,s);
2.60 (3H,s);
2.45 (3H,s).

### Preparation of 3-[2-(4-(methylthio)phenyl)acetyl](6-methyl)pyridine

A mixture of 8.0 g (28 mmol) of 3-[2-(4-(methylthio)phenyl)-2-cyanoacetyl](6-methyl)pyridine, 20 ml of acetic acid and 60 ml of concentrated hydrochloric acid was heated at 95°C to 100°C for 1.5 h.

The orange solution was cooled and adjusted to pH 10 using concentrated ammonia solution. The resulting yellow-beige suspension was filtered and the residue was washed with water and dried. This gave 5.35 g (74%) of the title product in the form of a yellow solid.
- ¹H-NMR (CDCl₃) :: 9.10 (1H,s);
8.15 (1H,d);
7.2 (5H,m);
4.21 (2H,s);
2.61 (3H,s);
2.45 (3H,s).

### Preparation of 1-(6-methylpyridin-3-yl)-2-[(4-(methylsulphonyl)phenyl]ethanone

Under an atmosphere of nitrogen, a suspension of 8.9 g (34.5 mmol) of 3-[2-(4-(methylthio)phenyl)acetyl](6-methyl)pyridine in 90 ml of methanol was heated to 55°C and adjusted to pH 4.5 using 2 N sulphuric acid. An aqueous solution of 0.22 g (0.7 mmol) of sodium tungstate in 7 ml of water was then added. At 55°C, 10 mol of hydrogen peroxide were then added over the course of 1 h, and the mixture was then cooled to room temperature and filtered. The slightly beige filtration residue was washed using 2 × 30 ml of a mixture of water/isopropanol 2:1 and 2 × 30 ml of water and then' dried under reduced pressure at room temperature. This gave 7.43 g of the title product in a yield of 75%.
- ¹H-NMR (CDCl₃) :: 9.15 (1H,s);
8.18 (1H,d);
7.92 (2H,d);
7.47 (2H, d) ;
7.30 (1H,d);
4.39 (2H,s);
3.04 (3H,s);
2.63 (3H,s).

## Claims

1. Process for preparing 1-(6-methylpyridin-3-yl)-2-[(4-(methylsulphonyl)phenyl]ethanone of the formula I 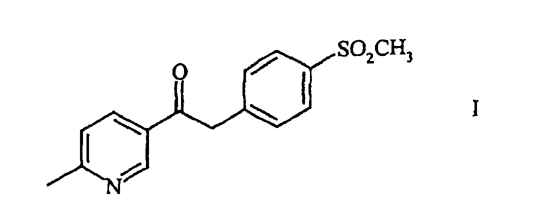 comprising the steps of:
conversion of 4-(methylthio)benzyl chloride in the presence of a phase transfer catalyst with an alkali metal cyanide in the presence of a water-immiscible solvent to give 4-(methylthio)phenylacetonitrile,
condensation of said 4-(methylthio)phenylacetonitrile with a 6-methylnicotinic ester to give 3-[2-(4-(methylthio)phenyl)-2-cyanoacetyl](6-methyl)pyridine of the formula II 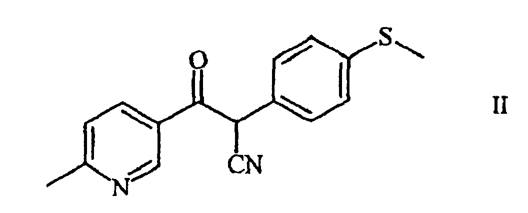
hydrolysis and decarboxylation of said 3-[2-(4-(methylthio)phenyl)-2-cyanoacetyl](6-methyl)pyridine of formula II under acidic conditions using a mixture of acetic acid and a mineral acid to give 3-[2-(4-(methylthio)phenyl)acetyl](6-methyl)pyridine of the formula III 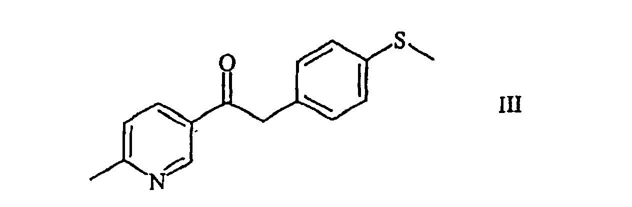
and, in the last step, oxidation of said 3-[2-(4-(methylthio)phenyl)acetyl](6-methyl)pyridine of formula III to give the end product.

2. Process according to Claim 1, **characterized in that** conversion of said 4-(methylthio)benzyl chloride is carried out at a temperature of from 60°C to 100°C.

3. Process according to Claim 1 or 2, **characterized in that** condensation of said 4-(methylthio)phenylacetonitrile is carried out in the presence of an alkali metal alkoxide at a temperature between 60°C and 110°C.

4. Process according to any one of Claims 1 to 3, **characterized in that** hydrolysis and decarboxylation are carried out at a temperature of from 50°C to 115°C.

5. Process according to any one of Claims 1 to 4, **characterized in that** oxidation in the last step is carried out using hydrogen peroxide in the presence of an alkali metal tungstate, at a temperature of from 10°C to 40°C.

6. Process according to any one of Claims 1 to 5, further comprising, as the first step, conversion of 4-(methylthio)benzyl alcohol with hydrochloric acid to give 4-(methylthio)benzyl chloride.

7. Process according to Claim 6, **characterized in that** conversion of 4-(methylthio)benzyl alcohol is carried out at a temperature of from 10°C to 40°C and in an organic solvent.

## Patentansprüche

1. Verfahren zur Herstellung von 1-(6-Methylpyridin-3-yl)-2-[4-(methylsulfonyl)phenyl]ethanon der Formel I 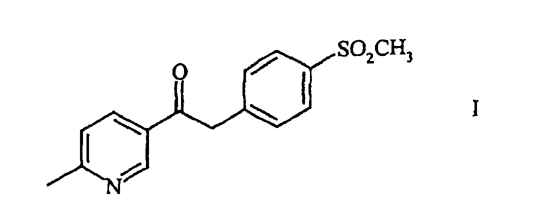 bei dem man:
4-(Methylthio)-benzylchlorid in Gegenwart eines Phasentransferkatalysators mit einem Alkalimetallcyanid in Gegenwart eines mit Wasser nicht mischbaren Lösungsmittels zu 4-(Methylthio)-phenylacetontril umsetzt,
das 4-(Methylthio)-phenylacetontril mit einem 6-Methylnicotinsäureester zu 3-[2-(4-(Methylthio)-phenyl)-2-cyanoacetyl]-6-methyl-pyridin der Formel II 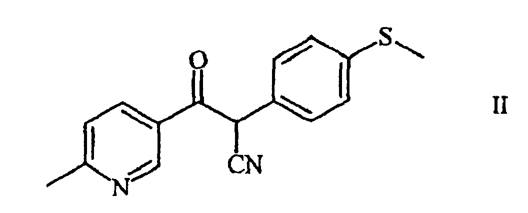 kondensiert,
das 3-[2-(4-(Methylthio)-phenyl)-2-cyanoacetyl]-6-methyl-pyridin der Formel II unter sauren Bedingungen mit einer Mischung aus Essigsäure und einer Mineralsäure zu 3-[2-(4-(Methylthio)-phenyl)acetyl]-6-methyl-pyridin der Formel III 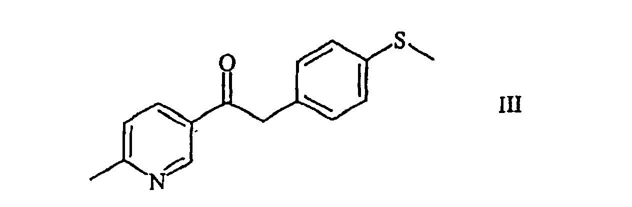 hydrolysiert und decarboxyliert
und im letzten Schritt das 3-[2-(4-(Methylthio)-phenyl)acetyl]-6-methyl-pyridin der Formel III zum Endprodukt oxidiert.

2. Verfahren nach Anspruch 1, **dadurch gekennzeichnet, daß** man die Umsetzung des 4-(Methylthio)-benzylchlorids bei einer Temperatur von 60°C bis 100°C durchführt.

3. Verfahren nach Anspruch 1 oder 2, **dadurch gekennzeichnet, daß**, daß man die Kondensation des 4-(Methylthio)-phenylacetonitrils in Gegenwart eines Alkalimetallalkoholats bei einer Temperatur zwischen 60°C und 110°C durchführt.

4. Verfahren nach einem der Ansprüche 1 bis 3, **dadurch gekennzeichnet, daß** man die Hydrolyse und Decarboxylierung bei einer Temperatur von 50°C bis 115°C durchführt.

5. Verfahren nach einem der Ansprüche 1 bis 4, **dadurch gekennzeichnet, daß** man die Oxidation im letzten Schritt mit Wasserstoffperoxid in Gegenwart eines Alkalimetallwolframats bei einer Temperatur von 10°C bis 40°C durchführt.

6. Verfahren nach einem der Ansprüche 1 bis 5, bei dem man ferner als ersten Schritt 4-(Methylthio)-benzylalkohol mit Salzsäure zu 4-(Methylthio)-benzylchlorid umsetzt.

7. Verfahren nach Anspruch 6, **dadurch gekennzeichnet, daß** man die Umsetzung von 4-(Methylthio)-benzylalkohol bei einer Temperatur von 10°C bis 40°C in einem organischen Lösungsmittel durchführt.

## Revendications

1. Procédé pour préparer la 1-(6-méthylpyridin-3-yl)-2-[(4-méthylsulfonyl)phényl]éthanone de formule I 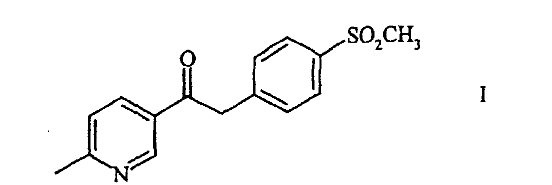 comprenant les étapes suivantes :
conversion de chlorure de 4-méthylthio-benzyle en présence d'un catalyseur de transfert de phase avec un cyanure de métal alcalin en présence d'un solvant non miscible à l'eau pour donner le 4-méthylthio)-phénylacétonitrile,
condensation dudit 4-méthylthio)phénylacétonitrile avec un ester 6-méthylnicotinique pour donner la 3-[2-(4-méthylthio)phényl)-2-cyanoacétyl]-6-méthyl-pyridine de formule II 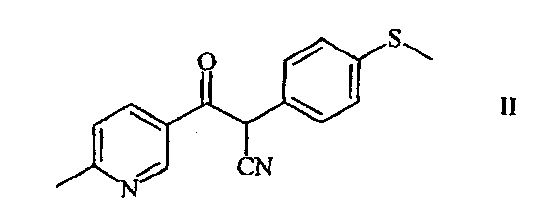
hydrolyse et décarboxylation de ladite 3-[2-(4-méthylthio)phényl)-2-cyanoacétyl]-6-méthyl-pyridine de formule II sous des conditions acides en utilisant un mélange d'acide acétique et d'un acide minéral pour donner la 3-[2-(4-méthylthio)phényl)acétyl]-6-méthylpyridine de formule III 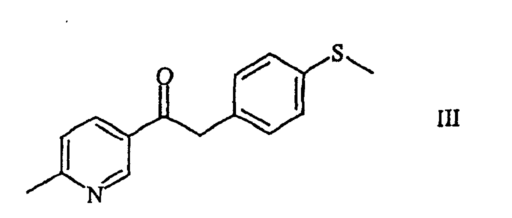
et, dans la dernière étape, oxydation de ladite 3-[2-(4-méthylthio)phényl)acétyl]-6-méthyl-pyridine de formule III pour donner le produit final.

2. Procédé selon la revendication 1, **caractérisé en ce que** la conversion dudit chlorure de 4-méthylthio-benzyle est effectuée à une température de 60°C à 100°C.

3. Procédé selon la revendication 1 ou 2, **caractérisé en ce que** la condensation dudit 4-méthylthio)phénylacétonitrile est effectuée en présence d'un alcoolate de métal alcalin à une température entre 60°C et 110°C.

4. Procédé selon l'une quelconque des revendications 1 à 3, **caractérisé en ce que** l'hydrolyse et la décarboxylation sont effectuées à une température de 50°C à 115°C.

5. Procédé selon l'une quelconque des revendications 1 à 4, **caractérisé en ce que** l'oxydation dans la dernière étape est effectuée en utilisant du peroxyde d'hydrogène en présence d'un tungstate de métal alcalin à une température de 10°C à 40°C.

6. Procédé selon l'une quelconque des revendications 1 à 5 comprenant en outre, comme première étape, la conversion d'alcool de 4-méthylthio-benzyle avec de l'acide chlorhydrique pour donner du chlorure de 4-méthylthio-benzyle.

7. Procédé selon la revendication 6, **caractérisé en ce que** la conversion de l'alcool de 4-méthylthio-benzyle est effectuée à une température de 10°C à 40°C et dans un solvant organique.
